# EUROPEAN PATENT APPLICATION

(11) **EP 4 527 381 A1**
(43) Date of publication of application: **26.03.2025**
(21) Application number: 23807656.6
(22) Date of filing: 17.05.2023
(51) Int. Cl.: A61K 31/12, A61K 31/282, A61P 13/12, A61P 35/00, A61K 33/243, A61K 45/06

(54) **PHARMACEUTICAL COMPOSITION FOR USE IN THERAPEUTIC OR PROPHYLACTIC METHOD FOR KIDNEY INJURY, COMBINED MEDICINE CONTAINING SAID PHARMACEUTICAL COMPOSITION, AND CCBL1 INHIBITOR**

(30) Priority: 20.05.2022 JP 2022083331
(71) Applicant: Fujita Academy, Toyoake-shi, Aichi 470-1192 (JP)
(72) Inventor: FUJIGAKI Hidetsugu, Toyoake-shi, Aichi 470-1192 (JP); SAITO Kuniaki, Toyoake-shi, Aichi 470-1192 (JP)
(74) Representative: Heyerhoff Geiger GmbH & Co. KG
(86) International application number: PCT/JP2023/018365
(87) International publication number: WO 2023/224055

(57) **Abstract**

An object is to provide a pharmaceutical composition for use in a treatment method or a prophylactic method for kidney injuries. The object can be achieved by a pharmaceutical composition for use in a treatment method or a prophylactic method for a kidney injury, the pharmaceutical composition includes a compound or a pharmaceutically acceptable salt of the compound as an active ingredient, the compound is expressed by the following general formula (1): 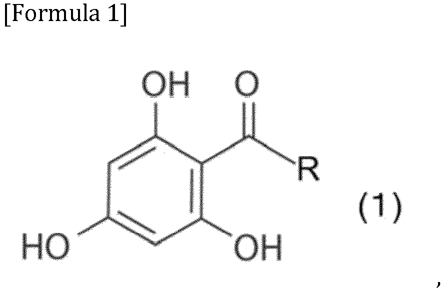 and
in formula (1), R represents a hydrogen atom, an alkyl group or alkenyl group having a straight or branched chain with 1 to 12 carbon atoms, an aralkyl group or arylalkenyl group with 7 to 12 carbon atoms that may have a substituent group, or an aromatic hydrocarbon group with 6 to 12 carbon atoms that may have a substituent group.

## Description

### [Technical Field]

The disclosure in the present application relates to a pharmaceutical composition for use in a treatment method or a prophylactic method for kidney injuries, a concomitant medication containing the pharmaceutical composition, and a CCBL1 inhibitor.

### [Background Art]

Platinum preparations are the most typical anticancer agents. Further, insurances are applied for platinum preparations used in a wide variety of cancers such as testicular tumors, bladder cancer, renal pelvis/ureter tumors, prostate cancer, ovarian cancer, head and neck cancer, non-small cell lung cancer, esophageal cancer, cervical cancer, neuroblastoma, gastric cancer, small cell lung cancer, osteosarcoma, germ cell tumors (testicular tumor, ovarian tumor, extragonadal tumor), malignant pleural mesothelioma, biliary tract cancer, malignant osteosarcoma, uterine cancer, relapsed/refractory malignant lymphoma, pediatric solid tumors (rhabdomyosarcoma, neuroblastoma, hepatoblastoma and other primary hepatic malignant tumors, medulloblastoma, and the like).

It is known, however, that platinum preparations may cause kidney injuries. The kidney injuries caused by platinum preparations affect decision as to whether or not to continue cancer treatment, the QOL of patients, or life prognosis. Further, in some patients, their kidney functions may have already decreased before cancer treatment is started. Thus, it is desired to develop a treatment method that can prevent the kidney function from decreasing due to administration of an anticancer agent or that enables safe implementation of treatment using an anticancer agent even for cancer patients whose kidney functions have already decreased.

To solve the above problem, Patent Literature 1 discloses that an agent capable of suppressing Interleukin-11 (IL-11)-mediated signaling can be used in a treatment method or a prophylactic method for cisplatin-induced kidney injuries.

### [Citation List]

### [Patent Literature]

Patent Literature 1: Japanese Patent Application Laid-Open No. 2022-521591

### [Summary of Invention]

### [Technical Problem]

The art disclosed above in Patent literature 1 is an art focusing on an action mechanism of suppressing Interleukin-11 (IL-11)-mediated signaling. Cells enable themselves to survive by making full use of various survival signals. It is thus desirable to develop a pharmaceutical composition that achieves an effect by different action mechanisms even for the same disorder (disease).

The disclosure in the present application has been made in order to solve the above problem. According to an intensive study made by the present inventors, it has been newly found that compounds represented by the following general formula (1) or pharmaceutically acceptable salts thereof can be used in a treatment method or a prophylactic method for kidney injuries and have a CCBL1 inhibitory function.

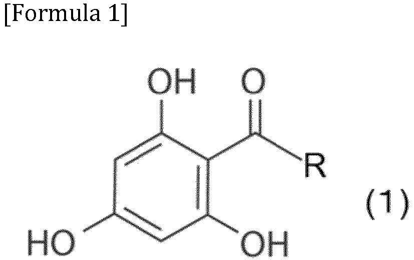

That is, an object of the disclosure in the present application is to provide a pharmaceutical composition for use in a treatment method or a prophylactic method for kidney injuries, a concomitant medication containing the pharmaceutical composition, and a CCBL1 inhibitor.

### [Solution to Problem]

The disclosure in the present application relates to a pharmaceutical composition for use in a treatment method or a prophylactic method for kidney injuries, a concomitant medication containing the pharmaceutical composition, and a CCBL1 inhibitor illustrated below.

[1] A pharmaceutical composition for use in a treatment method or a prophylactic method for a kidney injury, the pharmaceutical composition including a compound or a pharmaceutically acceptable salt of the compound as an active ingredient, and the compound being expressed by the following general formula (1): 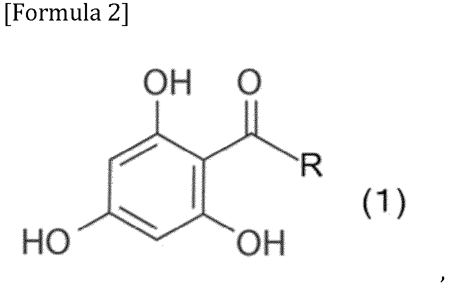 wherein in the formula (1), R represents a hydrogen atom, an alkyl group or alkenyl group having a straight or branched chain with 1 to 12 carbon atoms, an aralkyl group or arylalkenyl group with 7 to 12 carbon atoms that may have a substituent group, or an aromatic hydrocarbon group with 6 to 12 carbon atoms that may have a substituent group.
[2] The pharmaceutical composition according to [1] above, wherein the compound expressed by the general formula (1) has a CCBL1 inhibitory function.
[3] The pharmaceutical composition according to [1] above, wherein the general formula (1) is the following formula (2): 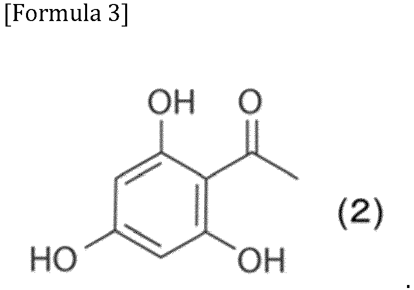
[4] The pharmaceutical composition according to [1] above, wherein the general formula (1) is the following formula (3): 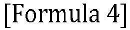 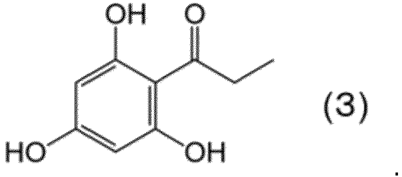
[5] The pharmaceutical composition according to [1] above, wherein the general formula (1) is the following formula (4): 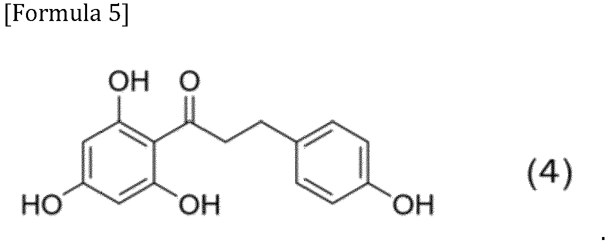
[6] The pharmaceutical composition according to any one of [1] to [5] above, wherein the kidney injury is an injury induced by administration of a platinum preparation.
[7] The pharmaceutical composition according to [6] above, wherein the kidney injury is a cisplatin-induced kidney injury.
[8] A concomitant medication including:
   a first medicine consisting of the pharmaceutical composition according to any one of [1] to [5] above; and
   a second medicine containing a kidney injury-inducible antineoplastic agent as an active ingredient.
[9] The concomitant medication according to [8] above, wherein the first medicine is administered before administration of the second medicine or at the same time as administration of the second medicine.
[10] A CCBL1 inhibitor including a compound or a pharmaceutical acceptable salt of the compound as an active ingredient, the compound being expressed by the following general formula (1): 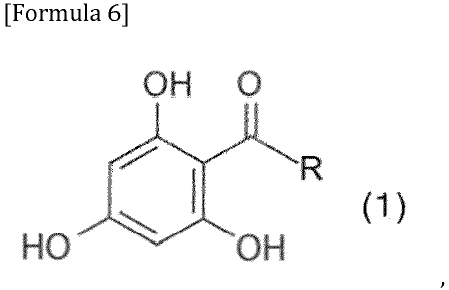 wherein in the formula (1), R represents a hydrogen atom, an alkyl group or alkenyl group having a straight or branched chain with 1 to 12 carbon atoms, an aralkyl group or arylalkenyl group with 7 to 12 carbon atoms that may have a substituent group, or an aromatic hydrocarbon group with 6 to 12 carbon atoms that may have a substituent group.
[11] The CCBL1 inhibitor according to [10] above, wherein the general formula (1) is the following formula (2): 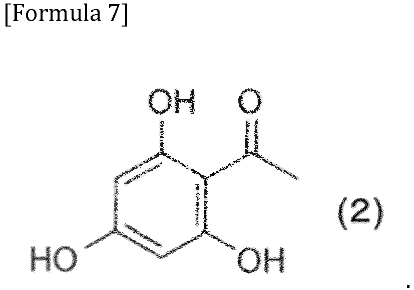
[12] The CCBL1 inhibitor according to [10] above, wherein the general formula (1) is the following formula (3): 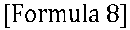 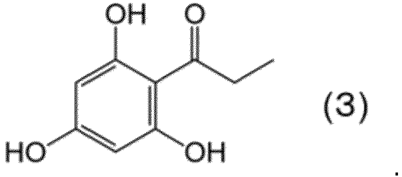
[13] The CCBL1 inhibitor according to [10] above, wherein the general formula (1) is the following formula (4): 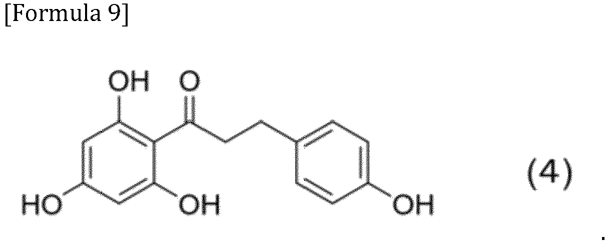
[14] The CCBL1 inhibitor according to any one of [10] to [13] used in a treatment method or a prophylactic method for a kidney injury.

### [Advantageous Effects of Invention]

The pharmaceutical composition disclosed in the present application can be used in a treatment method or a prophylactic method for kidney injuries. Further, the concomitant medication containing the pharmaceutical composition and a kidney injury-inducible antineoplastic agent can prevent antineoplastic agent-induced kidney injuries from occurring or, even when such an antineoplastic agent-induced kidney injury occurs, can reduce (treat) the degree of the injury. The compound represented by general formula (1) disclosed in the present application can be used as a CCBL1 inhibitor.

### [Brief Description of Drawings]

[FIG. 1]

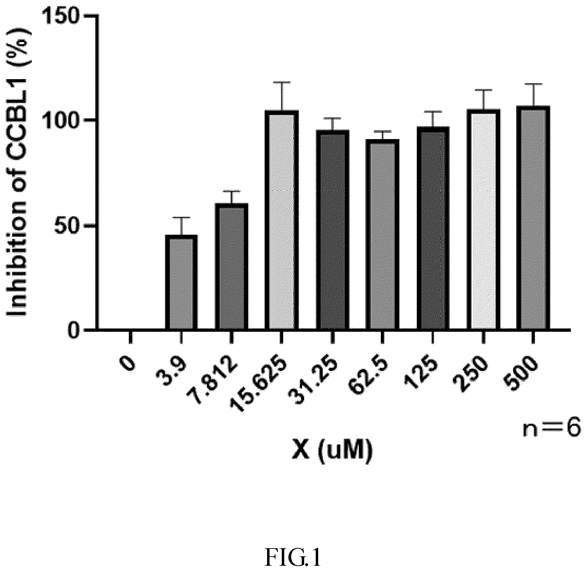

   FIG. 1 is a graph illustrating results obtained when inhibitory activity of a compound expressed by formula (2) to CCBL1β elimination activity is examined.
[FIG. 2]

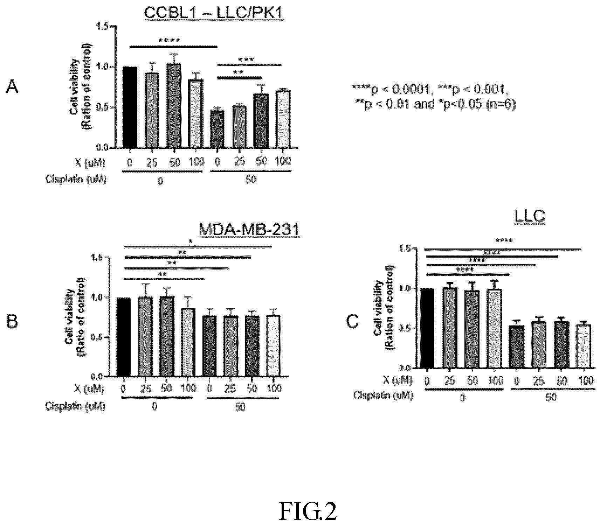

   FIG. 2 illustrates graphs illustrating results obtained when impacts of the compound expressed by formula (2) on renal tubular cells and cancer cells are examined. FIG. 2A illustrates a result with CCBL1 overexpressing LLC/PK1 cells, FIG. 2B illustrates a result with human breast cancer cell lines (MDA-MB-231 cells), and FIG. 2C illustrates a result with Lewis lung cancer cell lines (LLC cells).
[FIG. 3]

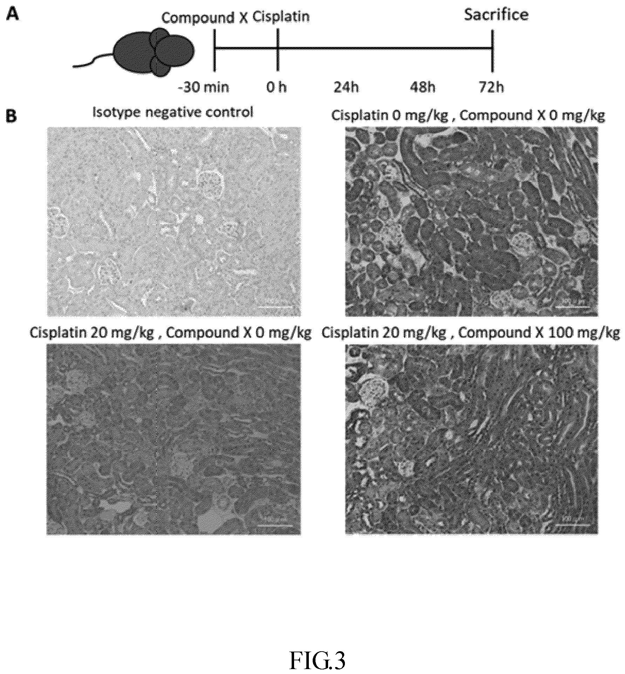

   FIG. 3 illustrates results obtained when impacts of the compound expressed by formula (2) on renal tubular cells and cancer cells are examined. FIG. 3A illustrates an experiment procedure. FIG. 3B includes photographs substitute for a drawing, which are photographs taken when amounts of CCBL1 protein expression in kidneys of respective groups are examined by immuno-histochemistry.
[FIG. 4]

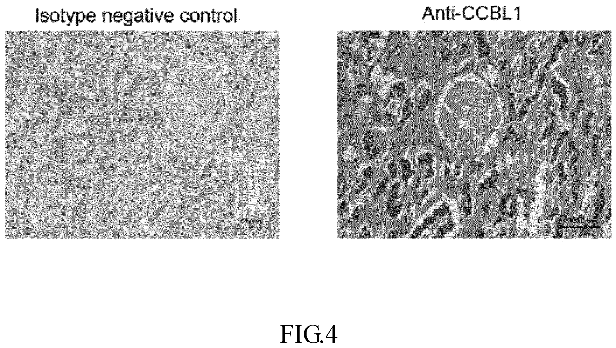

   FIG. 4 includes photographs substitute for a drawing, which are photographs indicating results of immune-stained human kidney tissues.
[FIG. 5]

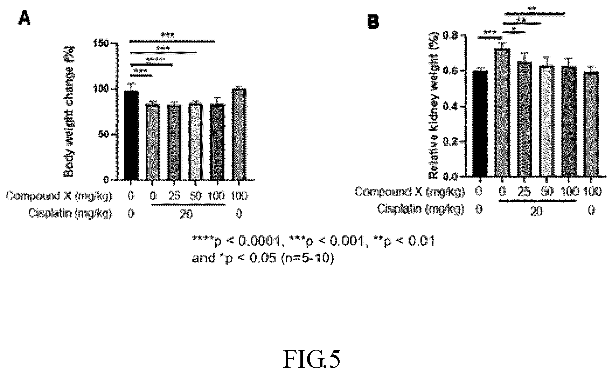

   FIG. 5 illustrates graphs obtained when impacts of the compound expressed by formula (2) on body weights and kidneys are examined, FIG. 5A illustrates the body weight change, and FIG. 5B illustrates the relative kidney weight.
[FIG. 6]

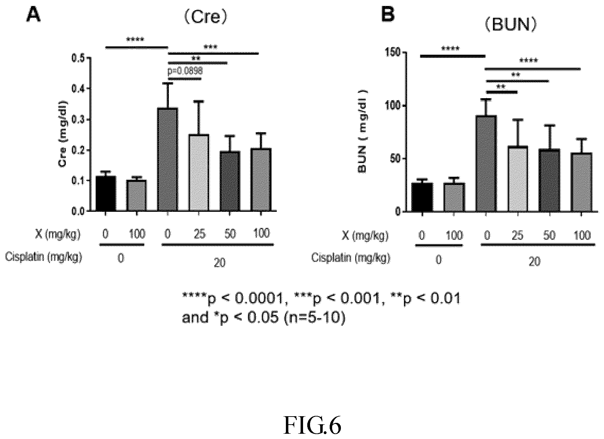

   FIG. 6 illustrates graphs illustrating results obtained when impacts of the compound expressed by formula (2) on kidney injuries are examined, FIG. 6A illustrates the Cre concentration, and FIG. 6B illustrates the BUN concentration.
[FIG. 7]

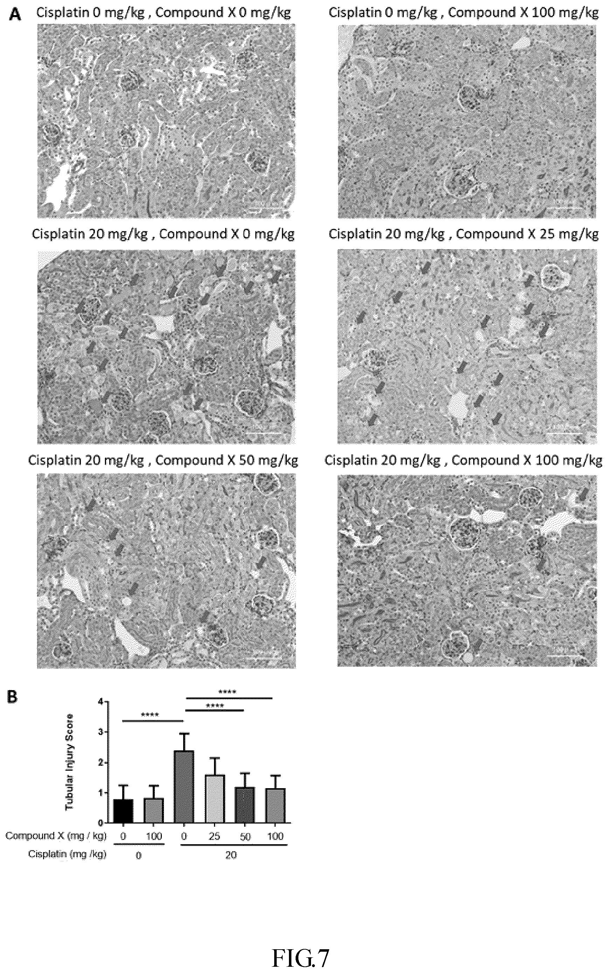

   FIG. 7 illustrates results of histopathological observation of mice administered with the compound expressed by formula (2). FIG. 7A includes photographs substitute for a drawing, which are optical micrographs. FIG. 7B is a graph illustrating the score.
[FIG. 8]

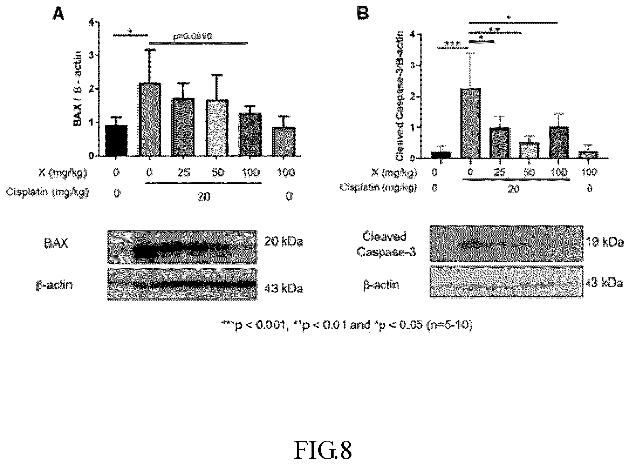

   FIG. 8 illustrates results obtained when inhibition of apoptosis in kidneys due to cisplatin achieved by the compound expressed by formula (2) is examined. FIG. 8A illustrates a result about BAX, and FIG. 8B illustrates a result about Cleaved Caspase-3.
[FIG. 9]

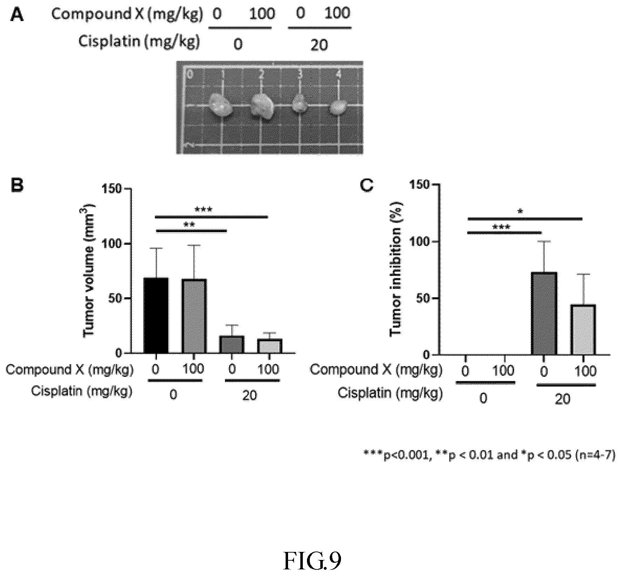

   FIG. 9 illustrates results obtained when the fact that the compound expressed by formula (2) does not adversely affect an anticancer action caused by cisplatin is examined. FIG. 9A is a photograph substitute for a drawing, which is a photograph of tumor tissues, FIG. 9B is a graph illustrating changes in the tumor volume, and FIG. 9C is a graph illustrating the tumor inhibition rate.
[FIG. 10]

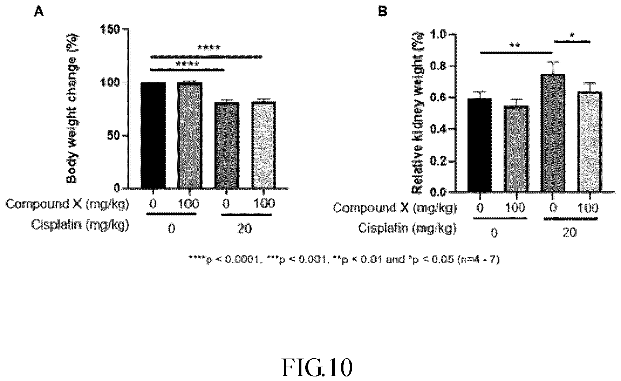

   FIG. 10 illustrates results obtained when the fact that the compound expressed by formula (2) does not adversely affect an anticancer action caused by cisplatin is examined. FIG. 10A is a graph illustrating the body weight change, and FIG. 10B is a graph illustrating the relative kidney weight.
[FIG. 11]

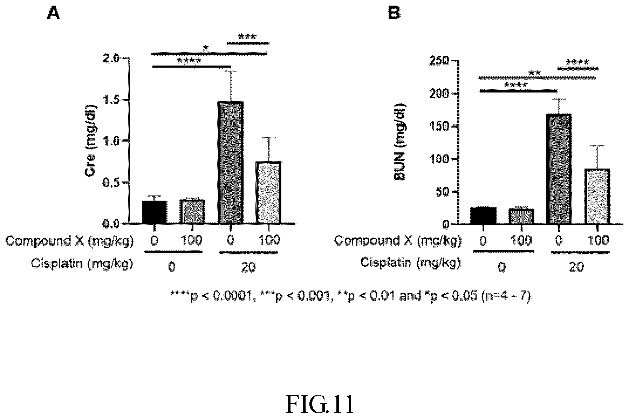

   FIG. 11 illustrates results obtained when the fact that the compound expressed by formula (2) does not adversely affect an anticancer action caused by cisplatin is examined. FIG. 11A is a graph illustrating the Cre concentration, and FIG. 11B is a graph illustrating the BUN concentration.
[FIG. 12]

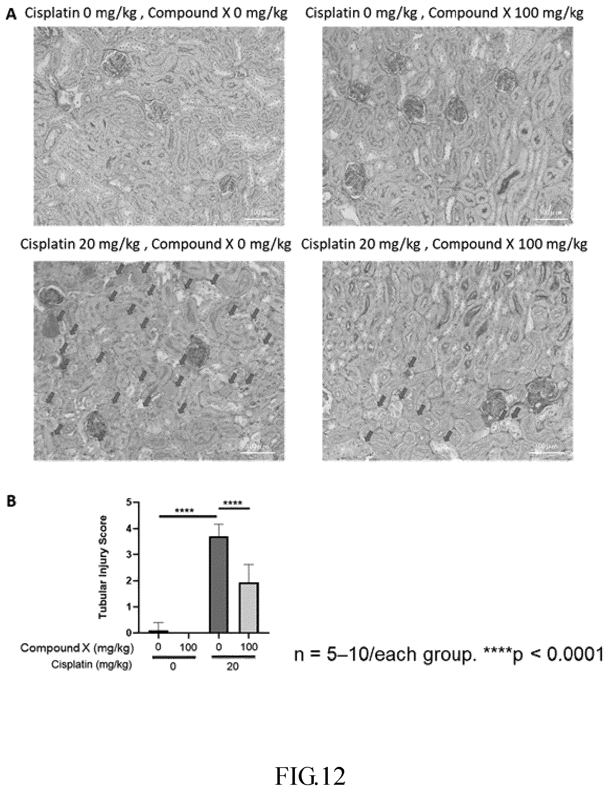

   FIG. 12 illustrates results obtained when the fact that the compound expressed by formula (2) does not adversely affect an anticancer action caused by cisplatin is examined. FIG. 12A includes photographs substitute for a drawing, which are optical micrographs. FIG. 12B is a graph illustrating the score.
[FIG. 13]

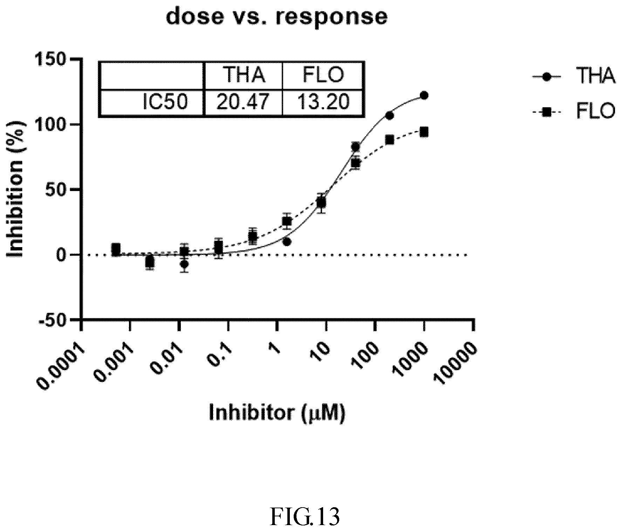

   FIG. 13 is a graph illustrating results obtained when inhibitory activity of a compound expressed by formula (3) to CCBL1β elimination activity is examined.
[FIG. 14]

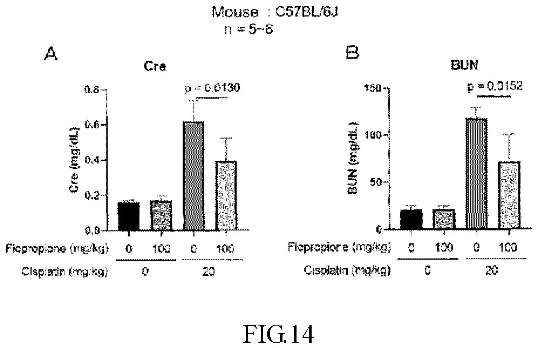

   FIG. 14 illustrates graphs illustrating results obtained when impacts of the compound expressed by formula (3) on kidney injuries are examined, FIG. 14A illustrates the Cre concentration, and FIG. 14B illustrates the BUN concentration.

### [Description of Embodiments]

A pharmaceutical composition for use in a treatment method or a prophylactic method (hereafter, which may be collectively referred to as "treatment method") for kidney injuries (hereafter, which may be simply referred to as "pharmaceutical composition"), a concomitant medication containing the pharmaceutical composition, and a CCBL1 inhibitor which are disclosed in the present application will be described below.

### <Embodiments of Pharmaceutical Composition>

The pharmaceutical composition contains a compound or a pharmaceutically acceptable salt thereof as an active ingredient, and the compound is expressed by the following general formula (1):

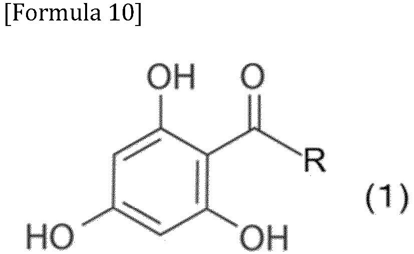

In general formula (1), R represents a hydrogen atom, an alkyl group or alkenyl group having a straight or branched chain with 1 to 12 carbon atoms, an aralkyl group or arylalkenyl group with 7 to 12 carbon atoms that may have a substituent group, or an aromatic hydrocarbon group with 6 to 12 carbon atoms that may have a substituent group.

The alkyl group or alkenyl group having a straight or branched chain with 1 to 12 carbon atoms may be, for example, a methyl group, an ethyl group, an n-propyl group, an isopropyl group, an n-butyl group, an isobutyl group, a sec-butyl group, a tert-butyl group, an n-pentyl group, an isopentyl group, a neopentyl group, a 1-methylpentyl group, an n-hexyl group, an isohexyl group, a vinyl group, an allyl group, a butenyl group, a hexenyl group, an n-heptyl group, a 2,4-dimethylpentyl group, a 1-n-propylbutyl group, an n-octyl group, a 2-ethylhexyl group, an n-nonyl group, a 1-methylnonyl group, an n-decyl group, a 3,7-dimethyloctyl group, a 2-isopropyl-5-methylhexyl group, an n-undecyl group, an n-dodecyl group, a decenyl group, or the like.

The aralkyl group or arylalkenyl group with 7 to 12 carbon atoms that may have a substituent group may be, for example, a benzyl group, a hydroxybenzyl group, a dihydroxybenzyl group, a phenylethyl group, a phenylethenyl group, a hydroxyphenylethyl group, a dihydroxyphenylethyl group, a hydroxyphenylethenyl group, a dihydroxyphenylethynyl group, a phenylpropyl group, a phenylpropenyl group, a phenylbutyl group, a phenylbutenyl group, a phenylpentyl group, a phenylpentenyl group, a phenylhexyl group, a phenylhexenyl group, or the like.

The aromatic hydrocarbon group with 6 to 12 carbon atoms that may have a substituent group may be, for example, a phenyl group, a hydroxyphenyl group, a dihydroxyphenyl group, a trihydroxyphenyl group, a naphthyl group, a hydroxynaphthyl group, a dihydroxynaphthyl group, or the like.

Note that the listing of R of general formula (1) is to describe those in a more preferable range and is not limited to the substituent groups illustrated above as examples as long as R is within a range that allows for the advantageous effect disclosed in the present application. Substituent groups that can be employed other than the substituent groups illustrated above as examples are listed below (for the purpose of convenience, the substituent groups listed below are defined as "R‴). The R' may be, for example, a hydroxy group, a halogen group such as F, a fluoroalkyl group such as -CF₃ or -CF₂CF₃, a cyclo lower alkyl group, a lower alkoxy group, a lower amino group, a trifluoromethoxy group, an amino lower alkyl group, a mono- or disubstituted amino lower alkyl group, a cyclic amino lower alkyl group, a lower alkenyl group, a styryl group, or the like. The cyclo lower alkyl group means a cyclo alkyl group with C3 to C8, which may be, for example, a cyclopropyl group, a cyclobutyl group, a cyclopentyl group, a cyclohexyl group, a cycloheptyl group, and a cyclooctyl group, and their rings may be substituted with an alkyl group having a straight or branched chain with C1 to C3, a hydroxy group, or a halogen atom.

Further, general formula (1) may be general formula (1') illustrated below.

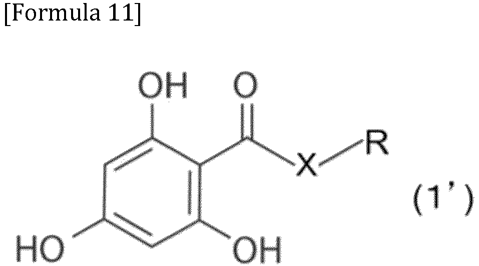

In general formula (1'), X represents CH₂, O, or NH. Further, R of general formula (1') is the same as R of general formula (1).

After creating an inhibitor screening system for enzymes involved in the onset of a cisplatin-induced kidney injury and performing screening tests on various compounds, the present inventors have revealed that compounds expressed by the above general formula (1) are useful. Specific compounds included in the above general formula (1) may be, for example, compounds expressed by the following formula (2), formula (3), and formula (4).

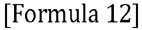

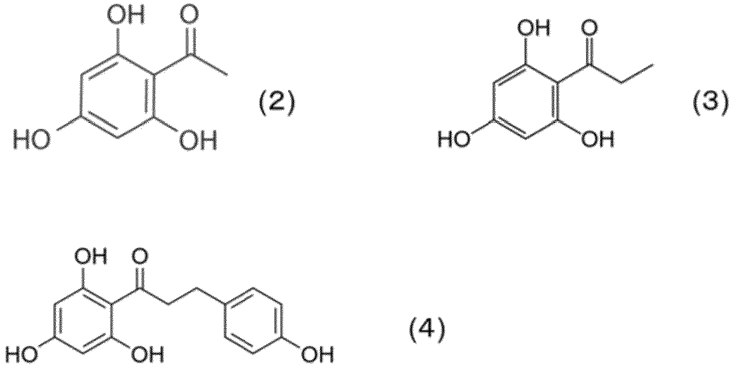

The above formula (2) represents a compound called 2,4,6-trihydroxyacetophenone (THA), which is also called PHLORACETOPHENONE. THA is a natural compound obtained from rhizomes of Curcuma comosa. THA is known as a matrix that is used in mass spectrometry using Matrix-Assisted Laser Desorption/Ionization (MALDI-TOF).

The above formula (3) represents a compound called 1-(2,4,6-trihydroxyphenyl)propan-1-one, which is also called FLOPROPIONE. FLOPROPIONE may be used as an antispasmodic agent. It is known that FLOPROPIONE administered to a human strongly acts on a particular site such as a biliary system around a duodenum, which may be administered targeting antispasmodic of the Oddi muscle, a bile duct, or a urinary tract. For example, FLOPROPIONE is administered against hepato-biliary-pancreatic disease for the purpose of facilitating bile from a bile duct and pancreatic juice from a pancreatic duct to flow into a duodenum. Similarly, when a urinary tract stone is present, FLOPROPIONE is administered for the purpose of facilitating the urinary tract stone to be excreted out of the body with urination.

The above formula (4) represents a compound called 3-(4-hydroxyphenyl)-1-(2,4,6-trihydroxyphenyl)propan-1-one, which is also called PHLORETIN. PHLORETIN is one of dihydrochalcone and polyphenol, which is a compound found in leaves of apple trees.

The compounds described above may be pharmaceutically acceptable salts thereof. The expression "pharmaceutically acceptable" means being useful in preparation of pharmaceutical compositions that are typically safe and nontoxic and are desirable biologically or in other respects and includes being acceptable for animal use and human pharmaceutical use. The pharmaceutically acceptable salts are not particularly limited as long as they are salts exhibiting desired pharmacological activity and may be, for example, inorganic acid salts such as hydrochloride, hydrobromide, sulfate, nitrate, oxalate, and phosphate and organic acid salts such as acetate, propionate, hexanoate, cyclopentanepropionate, glycolate, pyruvate, lactate, malonate, succinate, malate, fumarate, tartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, camphorsulfonate, 4-methylbicyclo[2.2.2]oct-2-ene -1-carboxylate, glucoheptanoate, 4,4'-methylenebis(3-hydroxy-2-ene-1-carboxylate), 3-phenylpropionate, trimethylacetate, tertiary butylacetate, lauryl sulfate, gluconate, glutamate, hydroxynaphthoate, salicylate, stearate, and muconate.

Further, the pharmaceutically acceptable salts of the compounds described above may be present as various solvates with, for example, methanol, ethanol, dimethylformamide, or the like.

The pharmaceutical composition can be locally administered or systemically administered. The administration form is not particularly limited, and the pharmaceutical composition can be orally administered or parenterally administered. Preparations for parenteral administration may contain a sterile aqueous or non-aqueous solution, suspension, emulsion, or the like. Examples of non-aqueous diluents may be propylene glycol, polyethylene glycol, vegetable oil, for example, olive oil, and organic ester compositions, for example, ethyl oleate or the like. An aqueous carrier may include any of water, alcoholic aqueous solutions, emulsions, suspensions, saline solutions, buffered media, and the like. A parenteral carrier may include any of sodium chloride solutions, Ringer's dextrose, dextrose, sodium chloride, Ringer's lactic acid, binding oil, and the like. An intravenous carrier may contain any of, for example, liquid supplements, nutrition, electrolytes (for example, those based on Ringer's dextrose), and the like.

The dosage form of the pharmaceutical composition may be, for example, a tablet, a pill, powder, a lozenge, a sachet, a cachet, an elixir, a suspension, an emulsion, a solution, a syrup, an aerosol agent (as a solid or in a liquid medium), an ointment, a gelatin soft and hard capsule, a suppository, a sterile injectable solution, sterile encapsulated powder, or the like.

As illustrated in Examples described later, when the pharmaceutical composition disclosed in the present application was administered before cisplatin was administered, improvement in values of creatinine (Cre) and blood urea nitrogen (BUN), which are indices of kidney functions, was found. Since the CCBL1 path is also involved in occurrence of drug (chemical substance)-induced kidney injuries due to other products derived from halogenated alkenes, the pharmaceutical composition disclosed in the present application is not limited to the use in treatment of platinum preparation (cisplatin)-induced kidney injuries caused by administration of platinum preparations such as cisplatin and is also useful for a platinum preparation uninvolved chemical substance-induced acute kidney injury, a chronic kidney injury, nephrotoxicity, or the like (hereafter, these may be collectively referred to as "platinum preparation uninvolved chemical substance-induced kidney injury"). Note that, in the present specification, "treatment method" means to treat platinum preparation-induced kidney injuries or platinum preparation uninvolved chemical substance-induced kidney injuries (hereafter, both the kidney injuries may be collectively referred to as "chemical substance-induced kidney injury"), and "prophylactic method" means to prevent chemical substance-induced kidney injuries from occurring. The platinum preparation may be cisplatin, carboplatin, oxaliplatin, or the like. The chemical substance other than platinum preparations that induces kidney injuries may be trichloroethylene, tetrafluoroethylene, busulfan, or the like. Note that the platinum preparation and the chemical substance other than platinum preparations that induces kidney injuries may be collectively referred to as "kidney injury-inducible chemical substance". Further, it is known that busulfan listed as an example of the chemical substance other than platinum preparations that induces kidney injuries is used as an antineoplastic agent. Platinum preparations or agents such as busulfan that achieve an antineoplastic effect but may induce a kidney injury may be referred to as a kidney injury-inducible antineoplastic agent. Further, a kidney injury induced by the kidney injury-inducible antineoplastic agent may be referred to as an antineoplastic agent-induced kidney injury.

As illustrated in the Examples described later, the present inventors have examined various types of activity of screened compounds and found that KAT1 (Cysteine Conjugate β-lyasel: CCBL1) inhibitory activity was observed in all the compounds of formula (2) to formula (4). It can be inferred that the kidney injury-use pharmaceutical compositions that have been newly found here act on the CCBL1 path and thereby achieve a treatment effect on kidney injuries.

The pharmaceutical composition disclosed in the present application can be used as follows, for example.
(1) To treat an antineoplastic agent-induced kidney injury, when occurred, or reduce the degree of the occurrence (prevention), administering the pharmaceutical composition to a patient before administration of a kidney injury-inducible antineoplastic agent,
(2) Administering the pharmaceutical composition at the same time as administration of a kidney injury-inducible antineoplastic agent,
(3) Administering the pharmaceutical composition when an antineoplastic agent-induced kidney injury occurs at a patient who has been administered with a kidney injury-inducible antineoplastic agent, or
(4) Administering the pharmaceutical composition to a patient in which the onset of a chemical substance-induced kidney injury (however, except for the antineoplastic agent-induced kidney injury) was confirmed.

### <Embodiment of Concomitant Medication>

Next, an embodiment of the concomitant medication will be described. The concomitant medication according to the embodiment contains a first medicine consisting of any pharmaceutical composition described in the embodiments of the pharmaceutical compositions and a second medicine containing a kidney injury-inducible antineoplastic agent as an active ingredient. The pharmaceutical composition, which is the first medicine, and the kidney injury-inducible antineoplastic agent contained in the second medicine have already been described in the above embodiment of the pharmaceutical composition. Thus, to avoid duplicated illustration, description of the pharmaceutical composition, which is the first medicine, and the kidney injury-inducible antineoplastic agent contained in the second medicine will be omitted.

When containing the kidney injury-inducible antineoplastic agent as an active ingredient, the second medicine can be of the same administration form, dosage form, or the like as the first medicine. Therefore, description of the administration form, the dosage form, or the like will be omitted to avoid duplicated illustration.

The first medicine contained in the concomitant medication is used for the purpose of preventing occurrence of an antineoplastic agent-induced kidney injury induced by the second medicine containing the kidney injury-inducible antineoplastic agent as an active ingredient or reducing the degree of the injury (treatment) even when the antineoplastic agent-induced kidney injury occurs. It is therefore desirable that the first medicine be administered before administration of the second medicine or at the same time as administration of the second medicine. The administration schedule and the dose can be determined by a physician based on a patient condition or the like.

### <Embodiment of CCBL1 Inhibitor>

Next, an embodiment of the CCBL1 inhibitor will be described. The CCBL1 inhibitor according to the embodiment contains the compound expressed by general formula (1) or a pharmaceutically acceptable salt thereof as an active ingredient as described above in <Embodiments of Pharmaceutical Composition>. As described above in <Embodiments of Pharmaceutical Composition>, the compound expressed by general formula (1) has KAT1 (Cysteine Conjugate β-lyase1: CCBL1) inhibitory activity. Therefore, the compound expressed by general formula (1) or the pharmaceutically acceptable salt thereof can be used as a CCBL1 inhibitor.

The CCBL1 inhibitor according to the embodiment is the same as the pharmaceutical composition for use in the treatment method or the prophylactic method for kidney injuries except that the compound expressed by general formula (1) or the pharmaceutically acceptable salt thereof is used as the CCBL1 inhibitor. Therefore, in the embodiment of the CCBL1 inhibitor, since "pharmaceutical composition" in the embodiment of the pharmaceutical composition can be replaced with "CCBL1 inhibitor", the detailed description of the embodiment of the CCBL1 inhibitor will be omitted.

Further, it can be inferred that the CCBL1 inhibitor acts on the CCBL1 path and thereby achieves a treatment effect on kidney injuries. Therefore, since the CCBL1 inhibitor can be used in the treatment method or the prophylactic method for kidney injuries, the CCBL1 inhibitor can also be used as the first medicine in the embodiment of the concomitant medication.

Although Examples will be presented below to specifically describe the embodiments disclosed in the present application, these Examples are for illustrative purposes only and are intended neither to limit the technical scope disclosed in the present application nor to express such limitation.

### [Examples]

### <Material>

### (1) Animal

All mice were accommodated in a plastic cage in which a 12-hour light/dark cycle (turned on at 8:00 A.M.) was maintained, and food and water were given (free intake). All experiments were performed in accordance with a guideline defined by Fujita Health University Animal Experiment Committee. The experimental protocol was approved by Fujita Health University Animal Experiment Committee (approval No: AP20004).

### (2) Cisplatin-induced kidney injury model mouse

To create cisplatin-induced kidney injury model mice, cisplatin (20 mg/kg) was administered once by intraperitoneal administration to male C57BL/6J mice with the body weight of 20 to 25 g (Japan SLC). To examine the kidney protection effect (prophylactic effect) of the compounds expressed by formulae (2) and (3) (hereafter, simply referred to as "compound") on a cisplatin-induced kidney injury, the compound was intraperitoneally administered with doses of 0, 25, 50, and 100 mg/kg 30 minutes before the cisplatin injection. The mice were sacrificed on the third day after the cisplatin injection. Samples of the blood and the kidneys were taken and preserved at -80 °C for further analysis.

### (3) Tumor allograft model mouse

Lewis lung cancer cells (1×10⁶ cells/50 µl PBS) were back-hypodermically injected to male C57BL/6J mice with the body weight of 20 to 25 g. Tumors were measured by using vernier calipers, and tumor volumes were calculated as a product of length by width by width. After the tumor reached a certain size, the compound (100 mg/kg) was intraperitoneally administered 30 minutes before the intraperitoneal administration of 20 mg/kg of cisplatin. The mice were sacrificed on the third day after the cisplatin injection. Samples of the blood, the tumors, and the kidneys were taken and preserved at -80 °C for further analysis.

### (4) Body weight change, kidney/body weight ratio, tumor inhibition

The body weight change (%), the kidney/body weight ratio, and the tumor inhibition were calculated by using the following equations. Body weight change (%) = [(body weight after treatment with cisplatin and the compound)/(body weight before administration of cisplatin and the compound X)] × 100 Relative kidney weight (%) = [kidney weight (g)/kidney weight (g) after treatment with cisplatin and the compound] × 100 Tumor inhibition (%) = value before administration of cisplatin and the compound - [(value after treatment with cisplatin and the compound X)/(value before administration of cisplatin and the compound)]

### (5) Cell culture

LLC/PK1 (pig kidney epithelium), LLC (Lewis lung cancer), and MDA-MB-231 (human breast cancer) cells were obtained from JCRB cell bank (Japan). The LLC/PK1 cells were transfected by a human CCBL1 expression vector, CCBL1 stable overexpression cells were cloned into CCBL1 overexpressed LLC/PK1 cells, which were used for further experiments. The CCBL1 overexpressed LLC/PK1 cells were proliferated in 199/EBSS culture medium (10% FBS, 1% penicillin/streptomycin, and 400 µg/ml of G418), and LLC and MDA-MB-231 cells were proliferated at 37 °C under humidified atmosphere of 5% CO₂ in DMEM culture medium (10% FBS and 1% penicillin/streptomycin).

### (6) Cell viability assay

A cell viability assay kit was used to measure the cell viability. Cells were seeded in a 96-well plate (2×10⁴ cells/well), treated with cisplatin and/or THA (the compound expressed by formula (2)) at different concentrations, and then cultured for 24 hours. Subsequently, 20 µL of CellTiter 96 aqueous solution (by Promega) was added to each well and cultured for another 1 hour. The absorbance at 490 nm was measured by using a microplate reader (Molecular Devices, USA). The cell viability was calculated as a value of the absorbance of treated cells relative to the absorbance of untreated cells.

### (7) Histopathological observation

A histopathological change of a kidney was examined by periodic acid-Schiff (PAS) stain. A half of the kidney was immersed and fixed in 10% buffered formalin and embedded in paraffin, and a resected segment was sliced thin into a thickness of 4 µm. After stained, the resected segment was observed by an optical microscope. Renal tubular damage was scored as follows. Each resected segment was observed in six fields of view, and the average ratio of damaged renal uriniferous tubules was observed. The result about damage of renal uriniferous tubules was converted into an index of renal uriniferous tubules necrosis, index 0 was allocated to an injury less than 10%, index 1 was allocated to an injury of 10% to 25%, index 2 was allocated to an injury of 25% to 50%, index 3 was allocated to an injury of 51% to 75%, and index 4 was allocated to an injury greater than 75%.

### (8) Immunostaining

Paraffin resected segments of human and mice were first dewaxed for 5 minutes in xylene (3 times) and subsequently substituted with fresh xylene. The obtained resected segments were treated for 2 minutes with ethanol anhydride and repeatedly treated with in the order of 90% ethanol for 2 minutes, 80% ethanol for 2 minutes, and 70% ethanol for 2 minutes. The deparaffinization-treated resected segments of the kidney were washed with water for 2 minutes. Endogenous peroxidase treatment (3% H₂O₂) was then performed for 20 minutes. The treated resected segments were then washed with distilled water for 2 minutes (twice) and with 1xPBS for 3 minutes (3 times). The resected segments were then blocked with 10% goat serum in 1xPBS for 20 minutes (CCBL1) or 30 minutes (B cell/CLL lymphoma 2; Bcl-2). The resected segments were incubated overnight at 4 °C together with the primary antibodies of anti-CCBL1 (1:500, GTX32492), anti-Bcl-2 (1:500, ab182858), and rabbit IgG iso-type control (1:500, ER1621011). On the next day, the resected segments were washed with 1xPBS for 3 minutes (3 times). Subsequently, slides were incubated together with the secondary antibody for 30 minutes (Histofine Simple Stain MAX PO; by NICHIREI BIOSCIENCES) and washed with 1xPBS for 3 minutes (3 times). Subsequently, the slides were incubated for 30 seconds together with a colorant/substrate reagent. Subsequently, these slides were nuclear-stained with hematoxylin. After stained, the resected segments were observed by an optical microscope.

### (9) Measurement of blood urea nitrogen and creatinine in serum

The levels of creatinine (Cre) and blood urea nitrogen (BUN) in serum were measured by using a fully automated blood biochemistry analyzer (BioMajesty JCA-BM9130) in accordance with a manufacturer-instructed method.

### (10) Western blotting

In immunoblotting analysis of protein and tissue lysates in human CCBL1 overexpressed LCC/PK1 cells, the protein and the tissue lysates were homogenized in a TNE buffer solution containing 10 mM Tris-HCl, 5 M NaCl at the final concentration of 0.15 M, 0.5 M EDTA at the final concentration of 1 mM, 1% NP40, and a protease inhibitor. The solution was centrifuged at 14,000 rpm at 4 °C for 10 minutes to obtain lysate supernatant. The total protein concentration in the lysate was determined by using Pierce 660 nm Protein assay kit (Thermo Scientific). The protein extracts were separated by sodium dodecyl sulfate-polyacrylamide gel electrophoresis (SDS-PAGE, 10% to 12.5% gels) and blotted onto membranes. After blocked with 5% fat-free milk, the membranes were incubated overnight together with anti-CCBL1 (1:1000, GTX32492, GeneTex), anti-Bcl-2 related X protein (BAX 1:5000, 50599-2-Ig, Thermo Fisher Scientific), anti-Cleaved Caspase-3 (1:2000, #9661, Abcam), and anti-β-actin (1:2000 or 1:5000, A5441, Santa Cruz), which are the primary antibodies. After washed with TBS-T, the membranes were incubated together with horseradish peroxidase-binding anti-IgG, and target bands were visualized with an enhanced chemiluminescence (ECL) reagent in accordance with the manufacturer's instruction. The intensities of the target bands were analyzed by densitometry and normalized with respect to β-actin.

### (11) Production of recombinant CCBL1 protein

Sf9 cells infected with baculovirus containing human CCBL1 gene were centrifuged and thereby pelletized and dissolved in 50 mM phosphate buffer solution (pH 8.0) containing 300 mM NaCl and 10 mM imidazole. After ultrasonic treatment, the cell lysate was centrifuged at 10,000 x g at 4 °C for 20 minutes, and recombinant CCBL1 in the supernatant was added to a pre-equalized Ni-NTA resin (QIAGEN). An enzyme/resin composite was transferred to a column and washed with a buffer solution containing 300 mM NaCl and 20 mM imidazole in 50 mM phosphate buffer solution (pH 8.0), and recombinant CCBL1 was eluted with a buffer solution (pH 8.0) containing 300 mM NaCl, 250 mM imidazole, and 50 mM phosphate. The enzyme fraction was pooled based on the purity determined by using SDS-PAGE and desalted by using a PD-10 column (GE Healthcare, U.K.)

### (12) Inhibition of CCBL1 activity by compound

The beta-elimination activity of CCBL1 was measured in accordance with a method described in "Arun Kumar Selvam et al., "A Novel Assay Method to Determine the -Elimination of Se-Methylselenocysteine to Monomethylselenol by Kynurenine Amino transferase 1" Antioxidants 2020, 9, 139; doi: 10. 3390/antiox 9020139". Briefly, 500 ng of recombinant CCBL1 protein in 5 µL and the compound at a different concentration in 1 µL were mixed, and the mixture was incubated at room temperature for 30 minutes. After the incubation, 44 µL of a reaction mixture contains 100 mM potassium phosphate buffer solution at pH 7.4, 5 mM Se-methylselenocysteine, 100 µM dimethyl-2-oxoglutarate, 100 µM α-keto-γ-methylthiobutyric acid sodium salt, 10 µM 2-amino-2-methyl-1,3-propanediol, and pyridoxal 5'-phosphoric acid hydrate. The reaction mixture was incubated at 37 °C, and the reaction was terminated by adding 2M HCl containing 20 µL of 5 mM 2,4-dinitrophenylhydrazine. Subsequently, 70 µL of the assay mixture was incubated at 37 °C for 10 minutes, 130 µL of 1M NaOH was added thereto, and the increase in the absorbance at 520 nm was measured by using a spectrophotometer (Nivo Multiplate Microplate Reader, PerkinElmer). The assay mixture containing no enzyme was used as a blank.

### (13) Data analysis

All the statistics analysis was performed by using GraphPad Prism 8 software (GraphPad Software Inc., San Diego, USA). The significant difference in comparison between two groups was evaluated by using one-way analysis of variance. The criteria for significance are as follows: ****p < 0.0001, ***p < 0.001, **p < 0.01, and *p < 0.05. All the data were represented as standard error ± mean.

### <Example 1>

The following experiments were performed by using THA expressed by formula (2). Note that THA used in Example 1 may be referred to as a compound X (simply "X" in the drawings).

### [In vitro experiment]

### (a) Inhibition of CCBL1 activity

To examine whether or not the compound X inhibits the CCBL1 enzyme activity, recombinant human CCBL1 was used to examine the inhibitory activity of the compound X relative to the CCBL1β elimination activity. FIG. 1 is a graph illustrating the results. As is clear from FIG. 1, it was confirmed that the CCBL1 activity was inhibited by the compound X in a concentration dependent manner.

### (b) Impact of compound X on renal tubular cell and cancer cell

To examine whether or not the compound X prevents cisplatin-induced renal uriniferous tubular cell death, CCBL1 overexpressed LLC/PK1 cells were treated with different concentrations of cisplatin and the compound X for 24 hours, and the cell viability was evaluated by cell proliferation assay. The expression amount of human CCBL1 in LLC-PK1 cells was determined by immunoblotting. FIG. 2 illustrates the results. As illustrated in FIG. 2A, the compound X did not affect the LLC-PK1 cell death in the group without addition of cisplatin (0 µM). On the other hand, the compound X prevented the cisplatin-induced renal tubular cell death in a dose dependent manner in the group with addition of cisplatin (50 µM). Next, FIG. 2B and FIG. 2C illustrate results obtained when human breast cancer cell lines (MDA-MB-231 cells) and Lewis lung cancer cell lines (LLC cells) were used to perform the same experiment. As is clear from FIG. 2B and FIG. 2C, the compound X did not affect the cisplatin-induced tumor cell death relative to the human breast cancer cell lines (MDA-MB-231 cells) and the Lewis lung cancer cell lines (LLC cells). From the results illustrated in FIG. 2A to FIG. 2C, it was confirmed that the compound X provides protection against cytotoxicity of cisplatin in renal uriniferous tubular cells without affecting the anticancer activity of cisplatin in cancer cells in vitro.

### [In vivo experiment]

### (c) Cisplatin-induced kidney injury in vivo caused by compound X

FIG. 3A illustrates the experiment procedure. As illustrated in FIG. 3A, to examine whether or not the compound X prevents the renal uriniferous tubular cell death in vivo, the compound X was injected to C57BL/6J mice 30 minutes before the cisplatin injection. Next, the amount of CCBL1 protein expression in kidneys in each group was examined by immuno-histochemistry. FIG. 3B illustrates the results. As is clear from FIG. 3B, CCBL1 mainly expressed in renal uriniferous tubular cells, and administration of cisplatin and the compound X did not affect the expression level of the CCBL1 protein.

FIG. 4 includes photographs indicating results of immune-stained human kidney tissues. As is clear from FIG. 4, it was confirmed that CCBL1 expressed in renal uriniferous tubular cells of human kidney resected segments.

### (d) Impact of compound X on body weight and kidney

Since it is known that administration of cisplatin reduces body weights and increases kidney weights, body weights and kidney weights of each group were measured. FIG. 5 illustrates the results. FIG. 5A illustrates the body weight change, and FIG. 5B illustrates the relative kidney weight. As is clear from FIG. 5A and FIG. 5B, administration of cisplatin alone reduced the body weight and increased the relative kidney weight. However, administration of both cisplatin and the compound X did not increase the relative kidney weight.

### (e) Impact of compound X on kidney injury

Next, the levels of kidney injuries were examined by measuring Cre and BUN in serum. FIG. 6 illustrates the results. FIG. 6A is a graph illustrating the Cre concentration, and FIG. 6B is a graph illustrating the BUN concentration. As is clear from FIG. 6, the administration of cisplatin alone increased the Cre and BUN levels. In contrast, although the administration of cisplatin increased the Cre and BUN levels, the administration of the compound X in addition to cisplatin reduces the Cre and BUN levels in a dose dependent manner. Further, when the compound X was administered alone, no change in the Cre and BUN levels was found.

### (f) Histopathological observation

Furthermore, renal uriniferous tubular cell injuries such as renal tubular cell death, cell dropout, interstitial edema, renal cortical tubular damage, or the like were examined by histopathological observation. FIG. 7 illustrates the results. FIG. 7A includes optical micrographs, and arrows in the photographs indicate injured cells. FIG. 7B is a graph illustrating the score. As is clear from FIG. 7, it was confirmed that the compound X suppresses the cisplatin-induced renal tubular cell death in a dose dependent manner. The group of cisplatin and the compound X had lower tubular injury scores than the group of only cisplatin. These results imply that the compound X prevents cisplatin-induced kidney injuries. Further, as is clear from the group to which only the compound X was administered, it was confirmed that the compound X itself does not injure renal uriniferous tubular cells.

### (g) Inhibition of apoptosis in kidney due to cisplatin achieved by compound X

It is known that cisplatin activates an apoptosis path of mitochondria (Shaomin Shi et al., "ER stress and autophagy are involved in the apoptosis induced by cisplatin in human lung cancer cells", Oncol rep, 2016; 2606-14.). Accordingly, the effect of administration of the compound X and cisplatin on apoptosis makers such as Bcl-2 related X protein (BAX) and Cleaved Caspase-3 was examined by immunoblotting in kidney samples resulted after administration of cisplatin and the compound X. FIG. 8A illustrates the result about BAX, and FIG. 8B illustrates the result about Cleaved Caspase-3. As is clear from FIG. 8A and FIG. 8B, the levels of BAX and Cleaved Caspase-3 decreased by the administration of the compound X in the group of administration of the compound X and cisplatin compared to the group of administration of cisplatin alone. From these results, it was confirmed that the compound X reduces apoptosis of renal uriniferous tubular cells due to cisplatin.

### (h) Compound X not adversely affecting anticancer action caused by cisplatin

It was examined whether or not the compound X can reduce renal uriniferous tubular cell damage without adversely affecting the anticancer action of cisplatin by using the tumor allograft model mice of (3) above. FIG. 9 to FIG. 12 illustrate the results. FIG. 9A is a photograph of tumor tissues, FIG. 9B is a graph illustrating the change in the tumor volume, and FIG. 9C is a graph illustrating the tumor inhibition rate. FIG. 10A is a graph illustrating the body weight change, and FIG. 10B is a graph illustrating the relative kidney weight. FIG. 11A is a graph illustrating the Cre concentration, and FIG. 11B is a graph illustrating the BUN concentration. FIG. 12A includes optical micrographs, and arrows in the photographs indicate injured cells. FIG. 12B is a graph illustrating the score. As is clear from FIG. 9A and FIG. 9B, administration of cisplatin reduced the tumor size, and this effect did not change even when the compound X was administered together with cisplatin. In the same manner as the case of the cisplatin-induced kidney injury model mouse, administration of cisplatin alone reduced the body weight and increased the relative kidney weight. As indicated in FIG. 10A and FIG. 10B, however, when both cisplatin and the compound X were administered, the body weight changed little, whereas the relative kidney weight decreased. Further, as indicated in FIG. 11A and FIG. 11B, the administration of the compound X reduced the increase in the cisplatin-induced BUN and Cre in a dose dependent manner. Furthermore, as is clear from FIG. 12A and FIG. 12B, it was confirmed that, in the tumor allograft model mice, the compound X reduces the renal uriniferous tubular damage score compared to the case of administration of cisplatin alone.

From the above results, it was confirmed that the compound X (THA) prevents cisplatin-induced renal uriniferous tubular cell damage (treats an injury that has occurred) both in vitro and in vivo. Further, it was confirmed that the compound X protects a kidney from a cisplatin-induced kidney injury without affecting an anticancer effect of cisplatin in vivo. Therefore, the compound X may be a potential adjuvant for clinical cisplatin therapy.

### <Example 2>

An experiment was performed in the same procedure as "(a) Inhibition of CCBL1 activity" and "(e) Impact of compound X on kidney injury" in Example 1 except that a compound expressed by formula (3) (flopropione) was used instead of THA of Example 1. FIG. 13 is a graph illustrating results of inhibition of CCBL1 activity. Note that, for comparison, FIG. 13 also illustrates the result about THA in addition. FIG. 14A is a graph illustrating the Cre concentration, and FIG. 14B is a graph illustrating the BUN concentration. As is clear from FIG. 13 and FIG. 14, it was confirmed that the CCBL1 activity is inhibited by flopropione in a concentration dependent manner in the same manner as in the case of THA. Further, while administration of cisplatin increased the Cre and BUN levels, flopropione reduced Cre and BUN in a dose dependent manner. Further, when flopropione was administered alone, no change was found in the Cre and BUN levels. From the above results, it was confirmed that flopropione achieves the same effect as THA.

### <Example 3>

### [Screening of CCBL1 inhibitory compound]

The CCBL1 inhibitory compound was obtained by screening KAT1 inhibitory activity of compounds included in a compound library (The Spectrum Collection) provided by Center for Supporting Drug Discovery and Life Science Research in Graduate School of Pharmaceutical Sciences, Osaka University. Here, 10 ng/µl recombinant CCBL, 1 mM L-kynurenine, 1 mM sodium pyruvate, 100 µM PLP, and 0.005% Tween 20 mixture solution contained in 150 mM of 2-amino-2-methyl-1-propanol buffer solution were added to a 384-well plate containing 10 µM of the compound and then incubated at room temperature for 2 hours. Subsequently, 20 µL of 300 mM zinc acetate solution was added thereto, and the fluorescent intensities at an excitation wavelength 340 nm and a fluorescent wavelength 460 nm were measured by Infinite M1000 plate reader (TECAN). The Control without containing the compound was defined as reaction 100%, the Background without containing any enzyme was defined as reaction 0%, and the inhibitory activity of the compound (Sample) was calculated based on the following equation. Inhibitory activity (%) = 100 × {1 - (Sample - Background)/(Control - Background)}

Table 1 lists the screening result of the compound. Note that the inhibitory activity of phloracetophenone used in Example 1 (the compound expressed by formula (2)) and that of flopropione used in Example 2 (the compound expressed by formula (3)) are listed together.

**[Table 1]**

| INHIBITORY ACTIVITY (%) | COMPOUND |
|---|---|
| 88.6 | PHLORACETOPHENONE |
| 71.9 | FLOPROPIONE |
| 83.0 | PHLORETIN |
| 5.6 | 4-O-METHYLPHLORACETOPHENONE |

As is clear from the result listed in Table 1, PHLORETIN (the compound expressed by formula (4)) also has a CCBL1 inhibitory function in the same manner as the compounds in Example 1 and Example 2 and therefore achieves the same effect as in Example 1 and Example 2.

In contrast, 4-O-methylphloracetophenone (having an antifungal function, see formula (5) below) in which 4-hydroxy of general formula (2) is substituted with 4-O-methyl exhibits little CCBL1 inhibitory activity despite the fact that 4-O-methylphloracetophenone is the same as general formula (2) except that the hydroxy group at position 4 of the benzene ring is substituted with an O-methyl group.

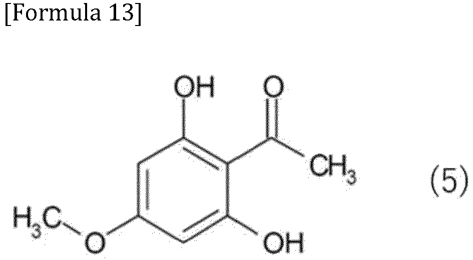

From the above results, since the compounds whose effects have been confirmed in Example 1 to Example 3 have the common backbone expressed by formula (1), any compounds that are expressed by formula (1) are expected to achieve the same advantageous effect. Further, since the compound expressed by formula (5) has little CCBL1 inhibitory activity, it can be inferred to be important that, in the compounds expressed by formula (1), the positions 2, 4, 6 of the benzene ring are a hydroxy group in order to achieve CCBL1 inhibitory activity.

### [Industrial Applicability]

The compounds expressed by formula (1) can reduce kidney injuries. Further, these compounds have a CCBL1 inhibitory function. It is therefore possible to perform treatment of cancer while suppressing kidney injuries induced by platinum preparations.

## Claims

1. A pharmaceutical composition for use in a treatment method or a prophylactic method for a kidney injury, the pharmaceutical composition comprising a compound or a pharmaceutically acceptable salt of the compound as an active ingredient, and the compound being expressed by the following general formula (1): 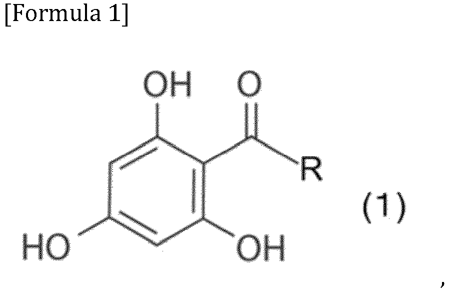 wherein in the formula (1), R represents a hydrogen atom, an alkyl group or alkenyl group having a straight or branched chain with 1 to 12 carbon atoms, an aralkyl group or arylalkenyl group with 7 to 12 carbon atoms that may have a substituent group, or an aromatic hydrocarbon group with 6 to 12 carbon atoms that may have a substituent group.

2. The pharmaceutical composition according to claim 1, wherein the compound expressed by the general formula (1) has a CCBL1 inhibitory function.

3. The pharmaceutical composition according to claim 1, wherein the general formula (1) is the following formula (2):

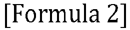

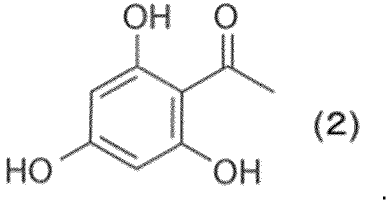

4. The pharmaceutical composition according to claim 1, wherein the general formula (1) is the following formula (3):

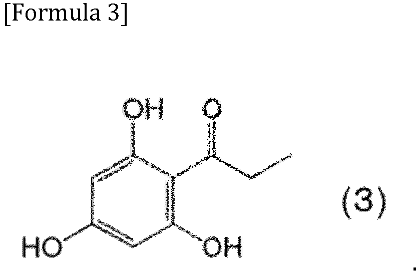

5. The pharmaceutical composition according to claim 1, wherein the general formula (1) is the following formula (4):

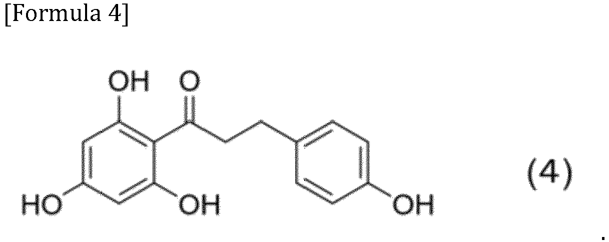

6. The pharmaceutical composition according to any one of claims 1 to 5, wherein the kidney injury is an injury induced by administration of a platinum preparation.

7. The pharmaceutical composition according to claim 6, wherein the kidney injury is a cisplatin-induced kidney injury.

8. A concomitant medication comprising:
a first medicine consisting of the pharmaceutical composition according to any one of claims 1 to 5; and
a second medicine containing a kidney injury-inducible antineoplastic agent as an active ingredient.

9. The concomitant medication according to claim 8, wherein the first medicine is administered before administration of the second medicine or at the same time as administration of the second medicine.

10. A CCBL1 inhibitor comprising a compound or a pharmaceutical acceptable salt of the compound as an active ingredient, the compound being expressed by the following general formula (1): 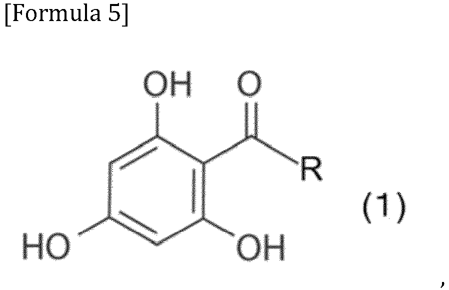 wherein in the formula (1), R represents a hydrogen atom, an alkyl group or alkenyl group having a straight or branched chain with 1 to 12 carbon atoms, an aralkyl group or arylalkenyl group with 7 to 12 carbon atoms that may have a substituent group, or an aromatic hydrocarbon group with 6 to 12 carbon atoms that may have a substituent group.

11. The CCBL1 inhibitor according to claim 10, wherein the general formula (1) is the following formula (2):

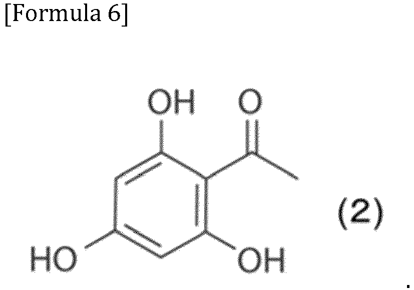

12. The CCBL1 inhibitor according to claim 10, wherein the general formula (1) is the following formula (3):

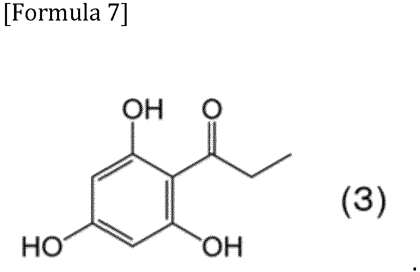

13. The CCBL1 inhibitor according to claim 10, wherein the general formula (1) is the following formula (4):

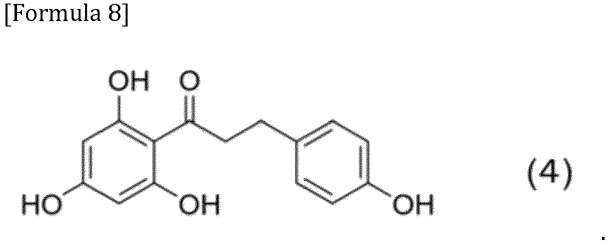

14. The CCBL1 inhibitor according to any one of claims 10 to 13 used in a treatment method or a prophylactic method for a kidney injury.
